# EUROPÄISCHE PATENTANMELDUNG

(11) **EP 3 495 017 A1**
(43) Veröffentlichungstag der Anmeldung: **12.06.2019**
(21) Anmeldenummer: 18210523.9
(22) Anmeldetag: 05.12.2018
(51) Int. Cl.: A61M 39/20

(54) **VERSCHLUSSVORRICHTUNG FÜR MEDIZINTECHNISCHE VERBINDUNGSSYSTEME**

(30) Priorität: 06.12.2017 DE 202017107432 U
(71) Anmelder: Hopf, Hans-Jürgen, 90513 Zirndorf (DE)
(72) Erfinder: HOPF, Michael, 90513 Zirndorf (DE); HOPF, Hans Jürgen, 90513 Zirndorf (DE); KASSAI, Norbert, 90522 Oberasbach (DE)
(74) Vertreter: Wittmann, Ernst-Ulrich

(57) **Zusammenfassung**

Die vorliegende Erfindung betrifft Verschlussvorrichtung (300) zu einem Verbindungssystem (10) an einem fluiddurchströmten Bauteil für die Medizin und/oder Medizintechnik, wobei das Verbindungssystem (10) einen oberen Teil (100) und einen unteren Teil (200) aufweist, wobei der obere Teil (100) des Verbindungssystems (10) mindestens eine Öffnung (110) zu einer oberen Fluidpassage (114), die sich in dem oberen Teil (100) erstreckt, aufweist, wobei der untere Teil (200) des Verbindungssystems (10) mindestens eine Öffnung (210) zu einer unteren Fluidpassage (214), die sich in dem unteren Teil (200) erstreckt, aufweist, wobei die obere Fluidpassage (114) mit der unteren Fluidpassage (214), vorzugsweise über ein Steuerungselement (400), verbunden ist, und die Verschlussvorrichtung (300) geeignet ist, die Öffnung (110) der oberen Fluidpassage (114) und/oder die Öffnung (210) der unteren Fluidpassage (214) zu verschließen. Die Erfindung ist dadurch gekennzeichnet, dass die Verschlussvorrichtung (300) mittels eines elastischen Bandes (360) an einem Befestigungsring (380), welcher mit dem oberen Teil (100) oder dem unteren Teil (200) des Verbindungssystems (10) verbunden ist, befestigt ist.

## Beschreibung

Die vorliegende Erfindung betrifft eine Verschlussvorrichtung zu einem Verbindungssystem an einem fluiddurchströmten Bauteil für die Medizin und/oder Medizintechnik.

Verschlussvorrichtungen für die Medizin und/oder Medizintechnik sind im Stand der Technik bekannt. Diese dienen dazu, fluiddurchströmte Bauteile gegen Verschmutzung und/oder Austrocknung zu schützen. Im Stand der Technik werden dafür Kappen oder ähnliche Vorrichtungen verwendet, welche entweder getrennt von dem Körper des Verbindungssystems sind oder an einem Punkt des Verbindungssystems fixiert sind.

Diese Vorrichtung weist unter anderem folgende Nachteile auf: Entweder können derartige lose Kappen leicht verloren gehen oder verschmutzt werden. Oder, im Fall der fixierten Kappen, können diese Vorrichtungen beim Handling des fluiddurchströmten Bauteils stören, indem sie z.B. in die Öffnung des Bauteils fallen.

Ausgehend von diesem Stand der Technik ist es Aufgabe der vorliegenden Erfindung, die Nachteile des Stands der Technik wenigstens teilweise zu überwinden bzw. zu verbessern.

Die Aufgabe wird durch eine Vorrichtung gemäß Anspruch 1 gelöst. Bevorzugte Ausführungsformen und Abwandlungen sind Gegenstand der Unteransprüche.

Eine erfindungsgemäße Verschlussvorrichtung zu einem Verbindungssystem an einem fluiddurchströmten Bauteil für die Medizin und/oder Medizintechnik, weist einen oberen Teil und einen unteren Teil auf. Dabei weist der obere Teil des Verbindungssystems mindestens eine Öffnung zu einer oberen Fluidpassage auf, die sich in dem oberen Teil erstreckt. Der untere Teil des Verbindungssystems weist mindestens eine Öffnung zu einer unteren Fluidpassage auf, die sich in dem unteren Teil erstreckt. Die obere Fluidpassage ist mit der unteren Fluidpassage, vorzugsweise über ein Steuerungselement, verbunden, und die Verschlussvorrichtung ist geeignet, die Öffnung der oberen Fluidpassage und/oder die Öffnung der unteren Fluidpassage zu verschließen.

Die Erfindung ist dadurch gekennzeichnet, dass die Verschlussvorrichtung mittels eines elastischen Bandes an einem Befestigungsring, welcher mit dem oberen Teil oder dem unteren Teil des Verbindungssystems verbunden ist, befestigt ist.

Ein Verbindungssystem an einem fluiddurchströmten Bauteil für die Medizin und/oder Medizintechnik liegt in einer Vielfalt von Formen vor. Einige dieser Verbindungssysteme weisen eine T-förmige oderY-förmige Gestalt auf, andere sind ohne Verzweigung ausgestaltet. Dementsprechend sind die Bezeichnungen "oberer Teil" und "unterer Teil" zu verstehen, nämlich als Bezeichnung eines ersten bzw. zweiten Teils eines Verbindungssystems. Bei einer zeichnerischen Darstellung- wie sie auch vorliegend gewählt ist - lässt sich dabei relativ klar ein "oben" und "unten" unterscheiden; im täglichen Gebrauch kann die Orientierung der Teile völlig unterschiedlich sein. Ein Verbindungssystem weist dabei sowohl "oben" als auch "unten" mindestens eine Öffnung auf, welche eine Fluidpassage, im Innern des Verbindungssystems, nach außen öffnet.

Vorzugsweise weist ein Verbindungssystem ein Steuerungselement auf, beispielsweise einen Hahn, welches das Öffnen, Schließen und/oder das teilweise Schließen mindestens einer Fluidpassage erlaubt. In anderen Ausführungsformen kann allerdings auf ein Steuerungselement verzichtet werden.

Eine erfindungsgemäße Verschlussvorrichtung erlaubt es nicht nur, mindestens eine der Öffnungen, mit einer der Öffnung angepassten Verschlussvorrichtung, zu verschließen. Darüber hinaus ist es möglich, die Verschlussvorrichtung mittels eines elastischen Bandes an einem Befestigungsring zu befestigen. Dabei ist der Befestigungsring mit dem oberen Teil oder dem unteren Teil des Verbindungssystems verbunden. Vorteilhafterweise ist dadurch die Verschlussvorrichtung verliersicher mit dem Verbindungssystem verbunden. Außerdem ist - durch die Anordnung eines elastischen Bandes an einem Befestigungsring - gewährleistet, dass die Verschlussvorrichtung nicht in störender Weise vor die Öffnung geraten kann. Dies verbessert das die Handhabbarkeit der Verschlussvorrichtung und führt zu einer weiteren Reduzierung des Risikos einer Verschmutzung der Verschlussvorrichtung und/oder der Öffnungen.

In einer Ausführungsform greift der Befestigungsring in eine umlaufende Nut ein, welche im Bereich des Randes der Öffnung angeordnet ist, so dass der Befestigungsring in Bezug auf den Rand der Öffnung drehbar ist.

Weil der Befestigungsring drehbar ist, wird die Handhabbarkeit der Verschlussvorrichtung weiter verbessert, weil somit die Verschlussvorrichtung, wenn sie geöffnet ist, wegen der Schwerkraft nach unten fällt und damit die Öffnung nicht in störender Weise verdeckt, aber jederzeit leicht greifbar ist. In einer Ausführungsform ist der Befestigungsring mit dem Rand der Öffnung, lösbar oder unlösbar, verbunden.

Der Befestigungsring kann auf die Öffnung der Fluidpassage gesteckt oder geklebt sein. In einer Ausführungsform kann der Befestigungsring auch einstückig mit dem Grundkörper der Öffnung hergestellt sein.

In einer Ausführungsform weist die Verschlussvorrichtung im Bereich ihrer Mitte einen Vorsprung auf, der geeignet ist, mindestens eine Öffnung der Fluidpassagen nach Art eines Pfropfens fluiddicht zu verschließen.

Diese Ausführungsform hat den Vorteil, dass damit der Halt der Verschlussvorrichtung in der Öffnung verbessert wird. Gleichzeitig wird die Dichtigkeit der Verschlussvorrichtung weiter erhöht.

In einer Ausführungsform ist der Vorsprung der Verschlussvorrichtung massiv ausgeführt. Dadurch wird die Steifigkeit der Verschlussvorrichtung erhöht.

In einer Ausführungsform weist der Vorsprung der Verschlussvorrichtung einen Hohlraum auf, welcher entweder in Richtung des Vorsprungs oder in Richtung der Verschlussvorrichtung offen ist. Dadurch ist der Vorsprung bei einem vorzugsweise verwendeten Material elastischer. Dies bewirkt, dass - wenn der Vorsprung nur einen geringfügig kleineren Durchmesser aufweist als die Öffnungsich der Vorsprung sehr eng in die Öffnung schmiegen kann und dadurch die Dichtigkeit der Verschlussvorrichtung weiter erhöht.

Für einen besonders dichten Verschluss der Öffnung kann der Vorsprung noch geriffelt sein, wobei der Vorsprung dann in entsprechende Vorsprünge bzw. Rücksprünge in der Fluidpassage eingreift. Diese Vorsprünge bzw. Rücksprünge können beispielsweise Wellenform oder die Form eines Sägezahns aufweisen.

In einer Ausführungsform ist das Verbindungssystem im Wesentlichen T-förmig oderY-förmig gestaltet, wobei der obere Teil zwei Öffnungen zu zwei verbundenen Fluidpassagen aufweist und der untere Teil über die untere Fluidpassage, vorzugsweise über ein Steuerungselement, mit mindestens einer Fluidpassage des oberen Teils verbunden ist. Diese Ausführungsformen werden zum Absperren mindestens einer Fluidpassage und/oder zur Auswahl zwischen mehreren Fluidpassagen verwendet.

In einer Ausführungsform weist die Verschlussvorrichtung im Bereich ihres Randes einen Wulst auf, der in dieselbe Richtung wie der Vorsprung weist und der geeignet ist, mindestens eine Öffnung der Fluidpassagen fluiddicht zu verschließen, indem, wenn die Verschlussvorrichtung mit der Öffnung verbunden ist, die Innenfläche des Wulstes mindestens teilweise den Rand der Öffnung umgreift.

Ein derartiger Wulst schützt außerdem die zur Öffnung weisende Seite der Verschlussvorrichtung davor, dass bei geschlossener Verschlussvorrichtung Staub oder andere Verunreinigungen zwischen die Öffnung und die Verschlussvorrichtung eindringen kann.

In einer Ausführungsform weist die Verschlussvorrichtung einen weiteren Wulst auf, der radial zwischen dem Vorsprung und dem Wulst angeordnet ist, der in dieselbe Richtung wie der Vorsprung weist und der geeignet ist, in eine weitere Nut einzugreifen, welche sich vom Rand der Öffnung, parallel zu der Öffnung, erstreckt.

Diese Ausführungsform ist insbesondere geeignet für Öffnungen, die eine zusätzliche vertikale Nut aufweisen, in welche der weitere Wulst eingreifen kann.

In einer Ausführungsform weist mindestens eine der Fluidpassagen ein Innengewinde auf. Das Innengewinde kann auch in einem getrennten Raum angeordnet sein, beispielsweise in einem Ringraum, der um die Fluidpassage angeordnet ist. Das Innengewinde kann entweder zylindrisch oder konisch gestaltet sein, so dass es auch für typische in der Medizintechnik verwendete Anschlussformen, wie z.B. für Luer-Anschüsse, geeignet sein kann.

In einer Ausführungsform ist an derVerschlussvorrichtung ein Griffelement angeordnet. Dies erleichtert das Öffnen derVerschlussvorrichtung. Dies ist insbesondere vorteilhaft für Ausführungsformen der Verschlussvorrichtung, die eine hohe Dichtigkeit bieten und daher oft eine höhere Kraft zum Öffnen bzw. Entfernen der Verschlussvorrichtung von der Öffnung benötigen.

In einer Ausführungsform ist die Verschlussvorrichtung (300) aus einem Material hergestellt ist, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, und/oder Kombinationen hiervon umfasst.

Die Erfindung wird nachfolgend anhand verschiedener Ausführungsformen erläutert, wobei darauf hingewiesen wird, dass durch dieses Beispiel Abwandlungen beziehungsweise Ergänzungen, wie sie sich für den Fachmann unmittelbar ergeben, mit umfasst sind. Darüber hinaus stellt dieses bevorzugte Ausführungsbeispiel keine Beschränkung der Erfindung in der Art dar, dass Abwandlungen und Ergänzungen im Umfang der vorliegenden Erfindung liegen.

Dabei zeigen:
- **Fig.1:**: Eine Schnittdarstellung eines Verbindungssystems mit einer erfindungsgemäßen Verschlussvorrichtung in perspektivischer Ansicht;
- **Fig. 2:**: ein Verbindungssystem mit geöffneter Verschlussvorrichtung in perspektivischer Ansicht von schräg unten;
- **Fig. 3:**: ein Verbindungssystem mit geschlossener Verschlussvorrichtung in perspektivischer Ansicht von schräg unten;
- **Fig. 4:**: eine Schnittdarstellung eines Verbindungssystems mit geschlossener Verschlussvorrichtung in Vorderansicht;
- **Fig. 5a bis 5d:**: Detailansichten einer alternativen Ausführungsform mit geschlossener Verschlussvorrichtung;
- **Fig. 6a bis 6d:**: Detailansichten einer alternativen Ausführungsform mit geöffneter Verschlussvorrichtung;

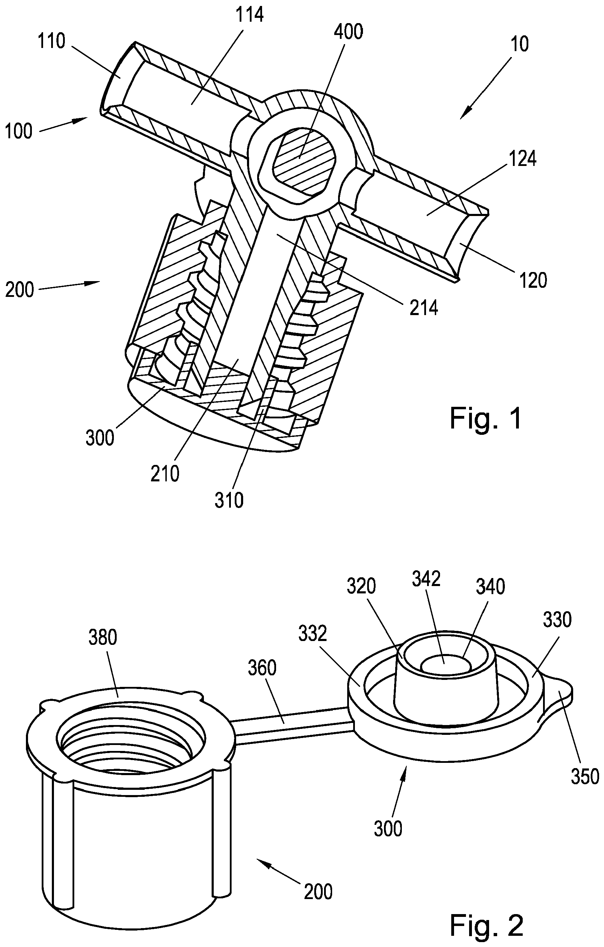

In **Fig. 1** ist in Schnittdarstellung ein Verbindungssystem 10 in T-förmiger Ausführung mit einer erfindungsgemäßen Verschlussvorrichtung 300, welche geschlossen ist, dargestellt. Dabei sind deutlich der obere Teil 100 und der untere Teil 200 des Verbindungssystems 10 zu erkennen. Der obere Teil 100 weist die Fluidpassage 114, 124 mit den jeweiligen Öffnungen 110, 120 auf. Etwa in der Mitte des oberen Teils 100 ist ein Steuerungselement 400 angeordnet (ebenfalls im Schnitt gezeigt), welches beispielsweise als Kükenhahn ausgeführt sein kann. Das Steuerungselement 400 kann, je nach Stellung, einen Durchlass zur Fluidpassage 214 des unteren Teils 200 öffnen. Die Fluidpassage 214 ist mittels der Verschlussvorrichtung 300 geschlossen. Die Verschlussvorrichtung 300 weist im Bereich ihrer Mitte einen Vorsprung 320 auf, der die Öffnung 210 der Fluidpassage 214 nach Art eines Pfropfens fluiddicht verschließt.

**Fig. 2** zeigt den unteren Teil 200 eines Verbindungssystems 10 mit geöffneter Verschlussvorrichtung 300 in perspektivischer Ansicht von schräg unten. Die Verschlussvorrichtung 300 ist mittels eines elastisches Bandes 360 verliersicher an dem Befestigungsring 380 mit dem Verbindungssystem 10 verbunden. Auf der rechten Seite der Verschlussvorrichtung 300 ist das Griffelement 350 erkennbar. Dies ist in Form einer Lasche ausgebildet, es kann aber auch als alternativ- wie im Stand der Technik bekannt - ausgestaltet sein. In der Mitte der Verschlussvorrichtung 300 ist ein Vorsprung 320 angeordnet. Dieser Vorsprung 320 weist einen Hohlraum auf und ist in Richtung des Vorsprungs 320 offen. Am Rande der Verschlussvorrichtung 300 ist ein Wulst 330 angeordnet, der in dieselbe Richtung wie der Vorsprung 320 weist. Zwischen dem Wulst 330 und dem Vorsprung 320 ist ein weiterer Wulst 340.

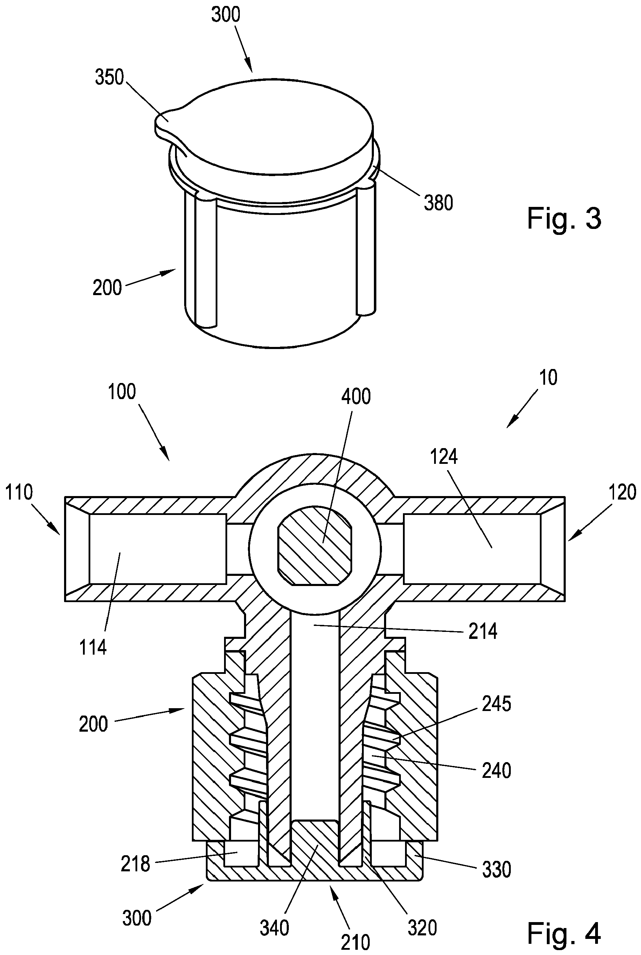

**Fig. 3** zeigt den unteren Teil 200 eines Verbindungssystems 10 mit geschlossener Verschlussvorrichtung 300 in perspektivischer Ansicht von schräg unten. Das Griffelement 350 ist auf der linken Seite erkennbar.

**Fig. 4** zeigt eine Schnittdarstellung durch ein Verbindungssystem 10 mit geschlossener Verschlussvorrichtung in Vorderansicht. Dabei ist deutlich das Zusammenwirken einer beispielhaften Verschlussvorrichtung 300 mit dem unteren Teil 200 eines Verbindungssystems 10 zu sehen. Es ist wieder ein T-förmiges Verbindungssystem 10 wie in Fig. 1 dargestellt. Der untere Teil 200 weist eine Fluidpassage 214 auf. Diese ist von einem ringförmigen Raum 240 umgeben. In diesem Ringraum 240 ist ein zylindrisches Innengewinde 245 angeordnet, zum Anschluss von medizinischen fluidführenden Geräten. Um sowohl die Fluidpassage 214 wie auch den Ringraum 240 zu schützen, wenn kein medizinisches Gerät angeschlossen ist, sind die Öffnungen von Fluidpassage 214 und Ringraum 240 durch die Verschlussvorrichtung 300 dicht abgeschlossen. Dazu ist am Rand der Verschlussvorrichtung 300 ein Wulst 330 angeordnet, der sich außen um die Öffnungen des unteren Teils 200 erstreckt und dadurch die Öffnungen des unteren Teils 200 verschließt. Um den Halt der Verschlussvorrichtung 300 in den Öffnungen weiter zu verbessern und um die Dichtigkeit derVerschlussvorrichtung 300 weiter zu erhöhen, sind weiterhin der Vorsprung 320 und der weitere Wulst 340 an der Verschlussvorrichtung 300 angeordnet. Der Vorsprung 320 greift dabei in die Fluidpassage 214 und der weitere Wulst 340 greift in eine weitere vertikale Nut 218 ein, welche sich vom Rand der Öffnung 210 parallel zu der Öffnung 210 erstreckt.

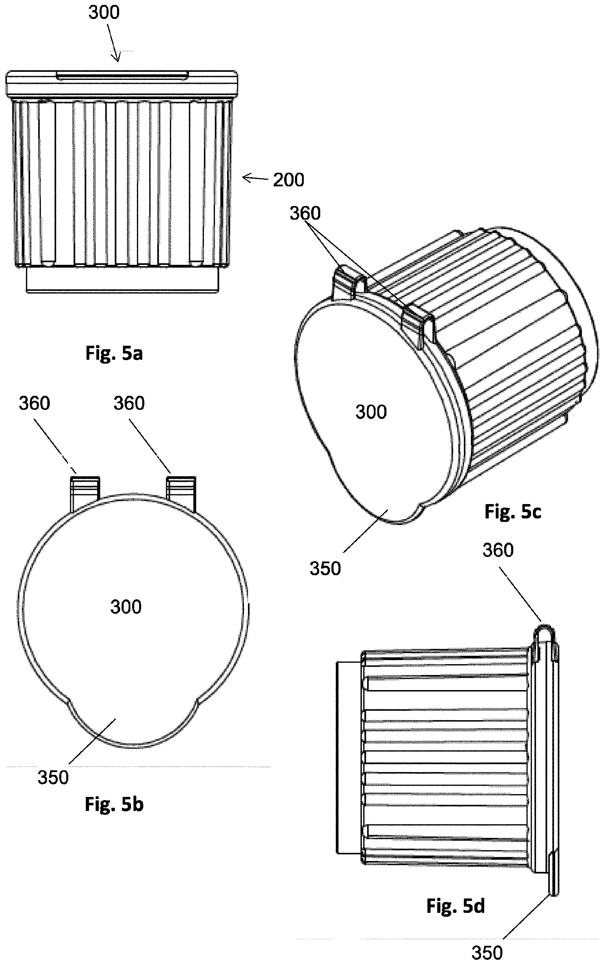

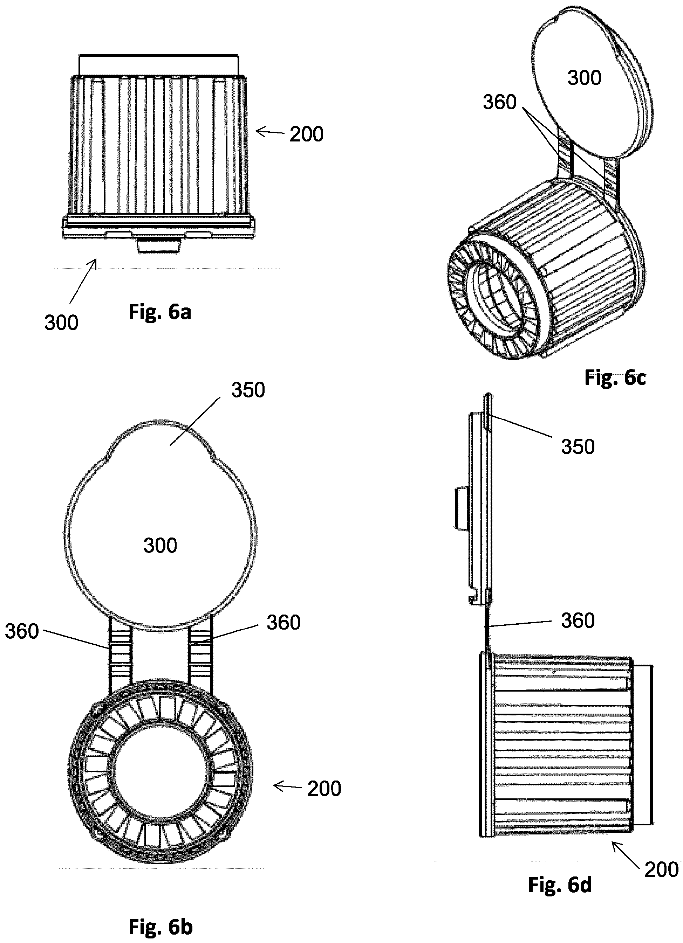

Die Figuren **5a bis 5d** zeigen Detailansichten einer alternativen Ausführungsform mit geschlossener Verschlussvorrichtung, bei welcher die Verschlussvorrichtung 300 mittels zweier Bänder 360, vorzugsweise zweier elastischer Bänder verliersicher und schwenkbeweglich mit dem unteren Teil 200 des Verbindungssystems verbunden ist. Die Figuren **6a bis 6d** zeigen Detailansichten der alternativen Ausführungsform aus den Figuren 5a bis 5d mit geöffneter Verschlussvorrichtung.

### Liste der Bezugszeichen

- 10: Verbindungssystem
- 100: oberer Teil
- 110: Öffnung
- 112: Rand der Öffnung
- 114: Fluidpassage
- 116: Nut
- 118: weitere Nut
- 120: Öffnung
- 122: Rand der Öffnung
- 124: Fluidpassage
- 126: Nut
- 128: weitere Nut
- 200: unterer Teil
- 210: Öffnung
- 212: Rand der Öffnung
- 214: Fluidpassage
- 216: Nut
- 218: weitere vertikale Nut
- 240: ringförmiger Raum, Ringraum
- 245: Innengewinde
- 300: Kappe/Verschlussvorrichtung
- 320: Vorsprung
- 330: Wulst
- 332: Innenfläche des Wulstes
- 340: weiterer Wulst
- 342: Innenfläche des weiteren Wulstes
- 350: Griffelement
- 360: elastisches Band
- 380: Befestigungsring
- 400: Steuerungselement

## Patentansprüche

1. Verschlussvorrichtung (300) zu einem Verbindungssystem (10) an einem fluiddurchströmten Bauteil für die Medizin und/oder Medizintechnik,
wobei das Verbindungssystem (10) einen oberen Teil (100) und einen unteren Teil (200) aufweist,
wobei der obere Teil (100) des Verbindungssystems (10) mindestens eine Öffnung (110) zu einer oberen Fluidpassage (114), die sich in dem oberen Teil (100) erstreckt, aufweist,
wobei der untere Teil (200) des Verbindungssystems (10) mindestens eine Öffnung (210) zu einer unteren Fluidpassage (214), die sich in dem unteren Teil (200) erstreckt, aufweist,
wobei die obere Fluidpassage (114) mit der unteren Fluidpassage (214), vorzugsweise über ein Steuerungselement (400), verbunden ist, und
die Verschlussvorrichtung (300) geeignet ist, die Öffnung (110) der oberen Fluidpassage (114) und/oder die Öffnung (210) der unteren Fluidpassage (214) zu verschließen,
**dadurch gekennzeichnet, dass**
die Verschlussvorrichtung (300) mittels eines elastischen Bandes (360) an einem Befestigungsring (380), welcher mit dem oberen Teil (100) oder dem unteren Teil (200) des Verbindungssystems (10) verbunden ist, befestigt ist.

2. Verschlussvorrichtung (300) nach Anspruch 1, **dadurch gekennzeichnet, dass**
der Befestigungsring (380) in eine umlaufende Nut (116, 126, 216) eingreift, welche im Bereich des Randes (112, 122, 212) der Öffnung (110, 120, 210) angeordnet ist, so dass der Befestigungsring (380) in Bezug auf den Rand (112, 122, 212) der Öffnung (110, 120, 210) drehbar ist.

3. Verschlussvorrichtung (300) nach Anspruch 1, **dadurch gekennzeichnet, dass**
der Befestigungsring (380) mit dem Rand (112, 122, 212) der Öffnung (110, 120, 210), lösbar oder unlösbar, verbunden ist.

4. Verschlussvorrichtung (300) nach Anspruch 1, 2 oder 3, **dadurch gekennzeichnet, dass**
die Verschlussvorrichtung (300) im Bereich ihrer Mitte einen Vorsprung (320) aufweist, der geeignet ist, mindestens eine Öffnung (110, 120, 210) der Fluidpassagen (114, 124, 214) nach Art eines Pfropfens fluiddicht zu verschließen.

5. Verschlussvorrichtung (300) nach Anspruch 4, **dadurch gekennzeichnet, dass**
der Vorsprung (320) derVerschlussvorrichtung (300) massiv ausgeführt ist.

6. Verschlussvorrichtung (300) nach Anspruch 4, **dadurch gekennzeichnet, dass**
der Vorsprung (320) derVerschlussvorrichtung (300) einen Hohlraum aufweist, welcher entweder in Richtung des Vorsprungs (301) oder in Richtung der Verschlussvorrichtung (300) offen ist.

7. Verschlussvorrichtung (300) nach Anspruch 1, **dadurch gekennzeichnet, dass**
das Verbindungssystem (10) im Wesentlichen T-förmig oder Y-förmig gestaltet ist, wobei der obere Teil (100) zwei Öffnungen (110, 120) zu zwei verbundenen Fluidpassagen (114, 124) aufweist,
und der untere Teil (200) über die untere Fluidpassage (214), vorzugsweise über ein Steuerungselement (400), mit mindestens einer Fluidpassage (114, 124) des oberen Teils (100) verbunden ist.

8. Verschlussvorrichtung (300) nach Anspruch 1, **dadurch gekennzeichnet, dass**
die Verschlussvorrichtung (300) im Bereich ihres Randes einen Wulst (330) aufweist, der geeignet ist, mindestens eine Öffnung (110, 120, 210) der Fluidpassagen (114, 124, 214) fluiddicht zu verschließen, indem, wenn die Verschlussvorrichtung (300) mit der Öffnung (110, 120, 210) verbunden ist, die Innenfläche (332) des Wulstes (330) mindestens teilweise den Rand (112, 122, 212) der Öffnung (110, 120, 210) umgreift.

9. Verschlussvorrichtung (300) nach Anspruch 8, **dadurch gekennzeichnet, dass**
die Verschlussvorrichtung (300) einen weiteren Wulst (340) aufweist, der radial zwischen dem Vorsprung (320) und dem Wulst (330) angeordnet ist, der in dieselbe Richtung wie der Vorsprung (320) weist und der geeignet ist, in eine weitere Nut (118, 128, 218) einzugreifen, welche sich vom Rand (112, 122, 212) der Öffnung (110, 120, 210), parallel zu der Öffnung (110, 120, 210), erstreckt.

10. Verschlussvorrichtung (300) nach einem der vorangehenden Ansprüche, **dadurch gekennzeichnet, dass**
mindestens eine der Fluidpassagen (114, 124, 214) ein Innengewinde aufweist.

11. Verschlussvorrichtung (300) nach einem der vorangehenden Ansprüche, **dadurch gekennzeichnet, dass**
an der Verschlussvorrichtung (300) ein Griffelement (350) angeordnet ist.

12. Verschlussvorrichtung (300) nach einem der vorangehenden Ansprüche, **dadurch gekennzeichnet, dass**
die Verschlussvorrichtung (300) aus einem Material hergestellt ist, das aus einer Gruppe ausgewählt ist, welche duro- und thermoplastische Kunststoff und insbesondere Polyphenylensulfid, Polypropylen, Poly-1-buten, Polyvinylchlorid, Polyvinylidenchlorid, Polymethyl-metaacrylat, Polymethylmethacrylat, Polyacrylnitril, Polystyrol, Polysulfon, Polyacetal, Polyvinylalkohol, Polyvinylacetat, Ethylen-Vinylacetat (EVA), Mischungen aus Ethylen-Vinylacetat und Polyethylen, Ethylen-Vinylacetat-Copolymer (EVAC) wie beispielsweise Elvax oder Evatane, lonomere, Fluorkunststoff, Polyethylen, Polyamid, insbesondere ein teilaromatisches Polyamid, Polycarbonat, Polyester, Polyphenylenoxid, Polysulfon, Polyvinylacetal, Polyurethan, und chlorierter Polyether, Zellulosenitrat, Zelluloseacetat, Zelluloseether, Phenol-Harz, Harnstoff-Harz, Thioharnstoff-Harz, Melamin-Harz, Alkylharz, Allylharz, Silicon, Polyimid, Polybenzimidazol, Epoxidharz, Casein-Kunststoff, vernetztes Polyurethan, ungesättigtes Polyesterharz, antimikrobielle oder antiseptische Materialen wie beispielsweise hochporöses Silber, ionenfrei hergestelltes Silber, Silberverbindungen und insbesondere Microsilber, Metallionen freisetzende Verbindungen, Bisphenol-A-freie Materialien wie zum Beispiel Tritan, Terlux, Acrylnitril-Butadien-Styrol, und/oder Kombinationen hiervon umfasst.
